# EUROPEAN PATENT APPLICATION

(11) **EP 3 488 701 A1**
(43) Date of publication of application: **29.05.2019**
(21) Application number: 17382803.9
(22) Date of filing: 27.11.2017
(51) Int. Cl.: A21D 6/00, A23L 7/10, A21D 13/047, A61K 8/00, A23L 2/58, D21H 21/28, A23L 5/20, A23L 19/00, A61K 8/97, A23C 11/00, A23G 3/48

(54) **RICE FLOURS AND USE THEREOF AS WHITENING ADDITIVES**

(71) Applicant: Herba Ingredients B.V., 1531 MG Wormer (NL)
(72) Inventor: PITTERY, Kristof, 1531 MG Wormer (NL); BARBER BENEDITO, Berta, 1531 MG Wormer (NL); BLANCO MUÑOZ, Miguel Ángel, 1531 MG Wormer (NL); GEERLINGS, Arjan, 1531 MG Wormer (NL)
(74) Representative: ABG Intellectual Property, S.L.

(57) **Abstract**

The invention relates to rice flours comprising specific amylose contents and particle size distributions, and to methods for preparing said rice flours. The rice flours of the invention can be employed as whitening additives in different fields of industry such as in paper, textile, plastic, paint, sealant, adhesive, concrete, food, fodder, beverage, cosmetic or pharmaceutical products.

## Description

### FIELD OF THE ART

The present invention relates to the field of pigments, and more particularly to the field of natural pigments, useful amongst others in food, cosmetic or pharmaceutical applications.

### BACKGROUND OF THE INVENTION

Titanium dioxide (TiO₂) is a naturally occurring oxide of titanium typically prepared from ilmenite (FeTiO₃) or titanium tetrachloride. TiO₂ is largely used as a pigment, and more specifically as a whitener, due to its very high refractive index. It can be employed in paints, coatings, plastics, paper, and also in foodstuffs, cosmetics or pharmaceuticals, e.g. in toothpastes, candies, or facial creams. Titanium dioxide accounts for 70% of the total production volume of pigments worldwide.

Although TiO₂ is generally recognized as safe (GRAS) by the U.S. Food and Drug Administration, recent studies are starting to point in the opposite direction. TiO₂ has now been classified by the International Agency for Research on Cancer (IARC) as an IARC Group 2B carcinogen which means it is "possibly carcinogenic to humans". In view of this suspected danger, a trend can be currently observed in industry, especially in the food industry, to develop more sustainable and healthy alternatives to TiO₂ in formulations.

Attempts so far to replace TiO₂ as whitening additive have mainly focused on employing alternative mineral materials. Amongst said materials calcium salts have received particular attention. For instance, US 2005/0152851 A1 discloses the use of calcium carbonate as whitener in dentifrices.

More recently, products have been developed based on organic materials, such as starches. For example, the company BENEO GmbH has disclosed a rice starch whitening product (Remy B7) suitable as a TiO₂ replacement.

It is an object of the present invention to provide a new type of whitener which can be useful as a replacement for TiO₂ in all or some of the above described industrial applications.

### SUMMARY OF THE INVENTION

The present inventors have now surprisingly found that specific rice flours can also act as whitening additive.

Despite the above mentioned known use of starches as TiO₂ replacement, the use of rice flours as whiteners has not to the knowledge of the inventors been suggested in the art.

Rice flour is unsuitable as a whitening agent as it contains a relatively high amount of protein, typically around 7-9%, in contrast to rice starch, which typically contains less than 1% protein. This high proportion of protein confers a yellowish tone to products comprising rice flour. Furthermore, darker tones tend to evolve upon exposure of these products to heat treatments usually employed in the different above mentioned industries.

The use of rice starches obviates these issues; however, the preparation of said starches requires a time- and cost-consuming purification process. Typically, acids and bases are used to precipitate the protein that is present in rice (7-9% protein). For this, the rice is first milled in the presence of water (wet milling) and once the protein is precipitated by the addition of e.g. acid, said protein is removed and separated from the starch. The remaining product is dried, and is called rice starch. Rice starch contains mainly starch, and less than 1% protein and fat. As a consequence of the starch purification process, many nutrients comprised in rice are lost.

The present inventors have now surprisingly found that specific rice flours can overcome all of the above mentioned issues. Firstly, the rice flours of the invention are white in appearance, and they do not undergo or minimally undergo browning upon heating. Secondly, rice flour has the same composition as rice, and therefore, its high nutritional values are minimally altered with respect to those of rice. Thirdly, the use of the rice flours does without the above laborious purification process required for the isolation of starch. The rice flours of the present invention are prepared by a production process which is simple and clean (no waste water production).

Thus, in a first aspect, the present invention provides a rice flour formed by rice flour particles, wherein rice flour particles having a size of between 10 and 100 µm represent at least 50% volume of the rice flour particles, and wherein the weight of amylose in the rice flour is at least 20% by weight with respect to the total weight of the rice flour.

In a second aspect, the present invention refers to a method for preparing a rice flour according to the first aspect of the invention, comprising the steps of:
a) selecting rice grains wherein the weight of amylose in the rice grains is at least 20% by weight with respect to the total weight of the rice grains;
b) reducing the particle size of the rice grains of step (a) until a rice flour formed by rice flour particles is obtained wherein rice flour particles having a size of between 10 and 100 µm represent at least 50% volume of the rice flour particles, and wherein the weight of amylose in the rice flour is at least 20% by weight with respect to the total weight of the rice flour.

In a third aspect, the present invention refers to a rice flour composition which comprises the rice flour of the first aspect of the invention.

In a further aspect, the invention relates to a paper, textile, plastic, paint, sealant, adhesive, concrete, food, fodder, beverage, cosmetic or pharmaceutical product comprising the rice flour of the first aspect of the invention or the rice flour composition of the third aspect of the invention.

In yet a further aspect, the present invention is directed to the use as a whitening additive of a rice flour of the first aspect of the invention or of a rice flour composition of the third aspect of the invention.

### BRIEF DESCRIPTION OF THE DRAWINGS

Figure 1 shows the particle size distribution of a rice flour prepared according to the method of the present invention.

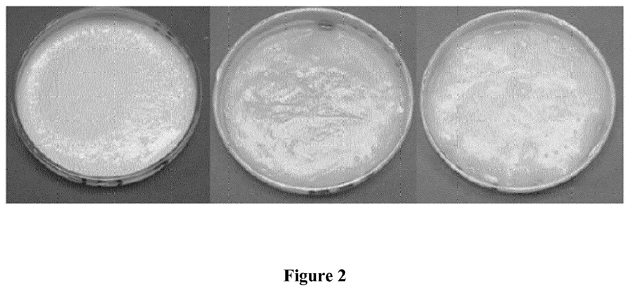

Figure 2 shows a side-to-side comparison of the whiteness of a titanium dioxide product (left), a rice starch product (middle) and a rice flour product according to the present invention (right).

### DETAILED DESCRIPTION OF THE INVENTION

The present invention refers to a rice flour formed by rice flour particles, wherein rice flour particles having a size of between 10 and 100 µm represent at least 50% volume of the rice flour particles, and wherein the weight of amylose in the rice flour is at least 20% by weight with respect to the total weight of the rice flour.

The term "rice flour" as used herein refers to particulate matter (e.g. powder) obtained from rice grain by comminution of the latter. Therefore, a rice flour is formed by or consists of rice flour particles.

In an embodiment, the rice flour is made from paddy, also known as paddy rice or rough rice. Paddy refers to rice retaining its husk after threshing.

In an embodiment, the rice flour is made from husked rice, also known as brown rice or cargo rice. Husked rice refers to paddy from which only the husk has been removed.

In an embodiment, the rice flour is made from milled rice, also known as white rice. Milled rice refers to husked rice from which the bran and embryo have been partly or fully removed, preferably by milling.

In a preferred embodiment, the rice flour is made from husked or milled rice. More preferably the rice flour is made from milled rice.

In an embodiment, the rice flour is made from non-boiled or non-parboiled rice.

The weight of amylose in the rice flours of the present invention is at least 20% by weight with respect to the total weight of the rice flour. Amylose is a helical polymer made of α-D-glucose units, bonded to each other through α(1→4) glycosidic bonds. The number of repeated glucose subunits (n) is usually in the range of 300 to 3000, but can be greater.

The inventors have surprisingly found that the yellow tone of rice flour which makes the flour unsuitable for use as a whitening additive, as well as the browning of said flour upon exposure of the flour to heat treatments (e.g. Maillard reaction), are greatly reduced when rice flour with specific amylose contents is employed.

In different embodiments, the weight of amylose in the rice flours of the present invention is at least 20% by weight amylose, at least 21% by weight amylose, at least 22% by weight amylose, at least 23% by weight amylose, at least 24% by weight amylose, at least 25% by weight amylose, at least 26% by weight amylose, at least 27% by weight amylose, at least 28% by weight amylose, at least 29% by weight amylose, or at least 30% by weight amylose, always with respect to the total weight of the rice flour.

In different embodiments, the weight of amylose in the rice flours of the present invention is up to 90% by weight amylose, up to 80% by weight amylose, up to 70% by weight amylose, up to 60% by weight amylose, up to 50% by weight amylose, up to 40% by weight amylose, up to 35% by weight amylose, up to 34% by weight amylose, up to 33% by weight amylose, up to 32% by weight amylose, up to 31% by weight amylose, always with respect to the total weight of the rice flour.

The immediately above low and high amylose content limits may be combined to provide close-ended amylose content ranges. In a preferred embodiment, the weight of amylose in the rice flours of the present invention is between 20% and 35% by weight with respect to the total weight of the rice flour. In another embodiment, the weight of amylose in the rice flours of the present invention is between 21% and 35% by weight with respect to the total weight of the rice flour. In another embodiment, the weight of amylose in the rice flours of the present invention is between 25% and 35% by weight with respect to the total weight of the rice flour.

In the context of the present invention, amylose content is determined according to ISO 6647-2:2007, Method B.

In an embodiment, the rice flour is prepared from non-genetically modified rice, or from genetically modified rice. In the context of the present invention, genetically modified rice refers to rice the amylose content of which has been increased by genetic engineering. Such genetically-modified rices are well-known in the art and are reported for instance in EP 1649022 B1.

In particular embodiment, the rice flour is prepared from genetically modified rice. In a preferred embodiment, the rice flour is prepared from non-genetically modified rice.

A vast number of natural rice varieties are known in the field of the invention. Non-limiting examples of rice varieties from which the rice flour of the present invention can be prepared are the Puntal; Doongara; Santa Anita; Micro 547 or 208; Cocodrie; El Paso; Don Juan; Tacuari; IRGA; Barbara; L-205; PSB Rc5, 10, 100 or 94; Paw San Mhwe or Bay Kyar; Paw San Yin; or Manawthukha varieties.

In a preferred embodiment, the rice flour is prepared from long-grain rice. Preferably, the long-grain rice is about 4 to 5 times long as it is wide. In a preferred embodiment, the rice flour is prepared from rice of the Indica variety. In a preferred embodiment, the rice flour is prepared from rice of the Puntal or Doongara varieties. In a particular embodiment, the rice flour is prepared from rice of the Doongara variety. In a preferred embodiment, the rice flour is prepared from rice of the Puntal variety.

In a preferred embodiment, when it is herein mentioned that the rice flour is made from particular rice, this means that the rice flour is made solely from said particular rice.

As described further below, when it is herein mentioned that the rice flour is made from particular rice, this means that the rice flour can be made from the grains of said particular rice, and also from flour prepared from said rice grains but which requires further processing to meet the requirements (e.g. particle size requirements) of the rice flours of the present invention.

In the rice flours of the present invention, rice flour particles having a size of between 10 and 100 µm represent at least 50% volume of the rice flour particles. In other words, the volume occupied by rice flour particles having a size of between 10 and 100 µm is at least 50% of the total volume occupied by all rice flour particles. The particle sizes and particle size distributions of the rice flours of the present invention can be attained by implementing the methods of preparation of the present invention described herein. The inventors have surprisingly found that the yellow tone of rice flour which makes the flour unsuitable for use as a whitening additive, as well as the browning of the flour upon exposure of the flour to heat treatments (e.g. Maillard reaction), are greatly reduced when the size of the rice flour particles is reduced to the extents herein described for the flours of the invention.

In different embodiments, at least 50% volume of the rice flour particles corresponds to rice flour particles having a size of between 20 and 100 µm, or between 20 and 95 µm, or between 20 and 90 µm, or between 20 and 75 µm, or between 20 and 50 µm, or between 20 and 30 µm, or between 30 and 100 µm, or between 30 and 95 µm, or between 30 and 90 µm, or between 30 and 75 µm, or between 30 and 50 µm; in a particular embodiment, between 20 and 50 µm; in a particular embodiment, between 30 and 50 µm; in a particular embodiment, between 20 and 75 µm; in a particular embodiment, between 30 and 75 µm.

In different embodiments, the immediately above particle sizes apply, but the volume percentage of particles with these sizes is at least 60%, at least 70%, at least 80%, or, preferably, at least 90%. In different embodiments, the immediately above particle sizes apply, but the volume percentage of particles with these sizes is between 50% and 97%, between 60% and 97%, between 70% and 97%, between 80% and 97%, or, preferably, between 90% and 97%. In different embodiments, the immediately above particle sizes apply, but the volume percentage of particles with these sizes is between 50% and 95%, between 60% and 95%, between 70% and 95%, between 80% and 95%, or, preferably, between 90% and 95%. In different embodiments, the immediately above particle sizes apply, but the volume percentage of particles with these sizes is between 50% and 91%, between 60% and 91%, between 70% and 91%, between 80% and 91%, or, preferably, between 90% and 91%. In different embodiments, the immediately above particle sizes apply, but the volume percentage of particles with these sizes is between 50% and 90%, between 60% and 90%, between 70% and 90%, or, preferably, between 80% and 90%. In different embodiments, the immediately above particle sizes apply, but the volume percentage of particles with these sizes is between 50% and 85%, between 60% and 85%, between 70% and 85%, or, preferably, between 80% and 85%.

Each of the above individual volume percent ranges can be combined with each of the above individual particle size ranges to arrive at additional embodiments.

In a preferred particular embodiment, rice flour particles having a size of between 20 and 50 µm, or of between 30 and 50 µm, represent at least 70%, preferably between 70% and 85% volume of the rice flour particles.

In a preferred particular embodiment, rice flour particles having a size of between 20 and 75 µm, or of between 30 and 75 µm, represent at least 70%, preferably between 70% and 97% volume of the rice flour particles.

In a preferred particular embodiment, rice flour particles having a size of between 20 and 50 µm, or of between 30 and 50 µm, represent at least 80%, preferably between 80% and 85% volume of the rice flour particles.

In a preferred particular embodiment, rice flour particles having a size of between 20 and 75 µm, or of between 30 and 75 µm, represent at least 80%, preferably between 80% and 97% volume of the rice flour particles.

In a preferred particular embodiment, rice flour particles having a size of between 20 and 75 µm, or of between 30 and 75 µm, represent at least 90%, preferably between 90% and 97% volume of the rice flour particles.

In the context of the present invention, particle size distribution is determined by laser diffraction analysis. More particularly, it is determined by laser diffraction analysis as set out in ISO 13320:2009 ((en): 2009-11).

During the comminution of rice grains, physical stress can break the starch comprised in the rice grain up into pieces and expose the internal amylose and amylopectin chains of the starch, rendering them liable to physical or enzymatic degradation. This alters the properties of the rice flour, generally leading to a reduction of the quality of the produced rice flour for the purposes of the present invention.

Thus, in a preferred embodiment, at least 80% of the starch in the rice flour of the invention is undamaged. In an embodiment, at least 85% of the starch is undamaged. In another embodiment, at least 90% of the starch is undamaged. In a preferred embodiment, at least 95% of the starch is undamaged. In a more preferred embodiment, at least 97% of the starch is undamaged.

In another embodiment, between 80% and 95% of the starch in the rice flour of the invention is undamaged. In an embodiment, between 85% and 95% of the starch is undamaged. In another embodiment, between 90% and 95% of the starch is undamaged.

In another embodiment, between 80% and 97% of the starch in the rice flour of the invention is undamaged. In an embodiment, between 85% and 97% of the starch is undamaged. In another embodiment, between 90% and 97% of the starch is undamaged. In another embodiment, between 95% and 97% of the starch is undamaged.

In the context of the present invention, starch damage is calculated according to AACCI (American Association of Cereal Chemists International) method 76-30.02. This method determines starch damage as g starch in a flour sample subject to fungal alpha-amylase hydrolysis per 100 g sample on a 14% moisture basis.

The skilled person knows how to prepare rice flour such that starch therein is undamaged. This is achieved by a combination of the grinding technology, the grinding parameters, and the quality of the raw material (rice grains) chosen.

The rice flours of the present invention can be prepared by a method comprising the following steps:
a) selecting a starting rice flour wherein the weight of amylose in said starting rice flour is at least 20% by weight with respect to the total weight of the starting rice flour;
b) reducing the particle size of the starting rice flour of step (a) until rice flour formed by rice flour particles is obtained wherein rice flour particles having a size of between 10 and 100 µm represent at least 50% volume of the rice flour particles, and wherein the weight of amylose in the rice flour is at least 20% by weight with respect to the total weight of the rice flour.

Alternatively, the rice flours of the present invention can be prepared by a method comprising the following steps:
a) selecting rice grains wherein the weight of amylose in the rice grains is at least 20% by weight with respect to the total weight of the rice grains;
b) reducing the particle size of the rice grains of step (a) until a rice flour formed by rice flour particles is obtained wherein rice flour particles having a size of between 10 and 100 µm represent at least 50% volume of the rice flour particles, and wherein the weight of amylose in the rice flour is at least 20% by weight with respect to the total weight of the rice flour.

Any of the embodiments of the first aspect of the invention can be prepared by a method of the second aspect of the invention. In order to arrive at said embodiments, the amylose content, and particle size and volume percentages chosen in the method of the invention are those described for the embodiment.

For instance, where the flour of the invention to be prepared comprises 30% by weight amylose with respect to the total weight of the rice flour, the starting rice flour and rice grains of step a) respectively comprise 30% by weight amylose with respect to the total weight of the starting rice flour and 30% by weight amylose with respect to the total weight of the rice grains. The percentage of amylose in the starting rice flour and rice grains of step a) does not necessarily need to exactly match the amylose percentage in the produced flour of the invention. An upper or lower tolerance, preferably of 3, more preferably of 1%, in the amylose amount of the starting rice flour and rice grains can also be employed in order to make up for any amylose weight increases or losses during particular preparation methods. In an embodiment, the tolerance is an upper tolerance. In another embodiment, the tolerance is a lower tolerance.

The rice varieties may be those mentioned for the flours of the invention. Either the rice grain or the rice flour of these grains can be selected for step a).

The size reduction of rice grains to produce conventional flours is generally achieved by milling the rice and passing the milled rice through a sieve of a known pore size, thus obtaining a flour with a maximum particle size corresponding to the pore size of the selected sieve. Typically, sieves of 250 micron are used so that flours are obtained with particles of the size of 250 micron or lower. Sieves of 150 micron may still be used for a rice flour, but sieves with smaller pore seizes are not suitable because they will get obstructed. Therefore, classical milling and sieving procedures cannot be employed for arriving at the particle sizes of the rice flours of the present invention.

In different embodiments, the particle size reduction of step b) of the methods of the invention is carried out until a flour with the particle size and particle size distribution of the flour of the invention as described in any of the above embodiments is obtained.

The size reduction of the rice grains or starting flour of step a) is preferably achieved by grinding, and more preferably by milling, and even more preferably by mechanical impact milling. The grinding or milling is preferably dry grinding or dry milling. Employing dry conditions is advantageous as it prevents the formation of waste water. This is in fact a problem encountered when purifying rice starch, which, as already described, typically requires wet milling.

In a preferred embodiment, the rice flour particle sizes and volume percentages of the rice flours of the present invention can be arrived at by a method of grinding rice grains or starting rice flour followed by air classification of the ground rice grains or starting rice flour. In a preferred embodiment, grinding and air classification are carried out simultaneously.

For this purpose, grinding and air classification are preferably carried out employing a combination mill, preferably an air classifying mill. These machines integrate the processes of grinding and air classification into a single circuit. In this type of apparatus, ground product is continuously discharged when it reaches the desired fineness, while material that is still too coarse continues to be ground. These mills apply high speed impact for size reduction and utilize a continuous internal recirculation of material to reduce oversize material which has not achieved the desired particle size. Classification is typically achieved using an integrated air classifier in the mill where forced air from below lifts ground material out of the grinding zone and circulates it to the classification zone. In the classification zone, particles smaller than the cut size of classification pass through the classifier and are then collected by a product collector. Oversize particles are sent back to the grinding zone. US 3285523 A describes a typical air classifying mill, whereas US 2002092938 A1 describes an air classifying mill with a varied configuration. The machines described by these documents are both suitable for preparing the flours of the present invention. The contents of these documents are therefore incorporated by reference in the present application.

The cut size of the air classifier is adjusted to obtain the desired particle size distribution. The person skilled in the art knows how to adjust the classifier cut size to obtain the herein described particle size distributions. A manner of obtaining the particle size distributions of the flours of the invention is to set the cut size of the air classifier to the size of the particles the highest proportion of which is sought, e.g. a flour wherein particles having a size of 30-50 µm represent a high volume of the rice flour can be achieved by setting the cut size of the air classifier to a value between 30-50 µm, preferably to lower values such that any oversize material may still be within the desired size range.

Step b) of the method of the invention can be carried out for as long as necessary to ensure achieving the desired flour particle size distribution. The time will vary depending upon the amount of rice to be ground or the desired target flour particle size distribution, higher amounts of rice and higher volumes of smaller particle sizes generally requiring longer times. In different embodiments, step b) is carried out for between 5 minutes and 10 hours, between 30 minutes and 5 hours, or between 1 hour and 3 hours.

The method of the present invention optionally comprises a further step c) wherein the flour obtained in step b) is subjected to heating, preferably dry heating. The present inventors have found that amylase enzymatic activity can be inactivated when the flours of the invention are subjected to a heat treatment, thus allowing for long-term storage of the flours prior to use without any or with low amylose loss. The inventors have further found that the size of the rice flour particles can be further lowered by such heat treatment.

In different embodiments, the heating is carried out for at least 1 minute, at least 5 minutes or at least 15 minutes. In different embodiments, the heating is carried out for at most 1 hour, at most 30 minutes or at most 15 minutes. These minimum or maximum values can be combined to generate time ranges. In an embodiment, heating is performed for a time of between 1 and 15 minutes.

In different embodiments, the heating is carried out at a temperature of at least 40 °C, at least 70 °C, or at least 110 °C. In different embodiments, the heating is carried out at a temperature of at most 200 °C, at most 150 °C or at most 110 °C. These minimum or maximum values can be combined to generate temperature ranges. In a preferred embodiment, the heating is carried out at a temperature of between 40 °C and 150 °C. In a particular embodiment, the heating is carried out at a temperature of between 40 °C and 110 °C. In another particular embodiment, the heating is carried out at a temperature of between 70 °C and 150 °C.

These heating temperatures can be combined with the above heating times. As a general rule, higher temperatures require lower times. In an embodiment, heating is performed at a temperature of between 40 °C and 150 °C for a time of between 1 and 15 minutes. In a particular embodiment, heating is performed at a temperature of between 40 °C and 110 °C for a time of between 1 and 15 minutes. In another particular embodiment, heating is performed at a temperature of between 70 °C and 150 °C for a time of between 1 and 15 minutes.

The use of dry conditions for the milling and/or heating of the rice flour of the present invention is preferred in the context of the present invention.

In another aspect, the present invention refers to a rice flour obtainable by a method according to the present invention in any of the above described embodiments.

The present invention also refers to a rice flour composition comprising or consisting essentially of the rice flour of the present invention. The rice flour composition may for example include, in addition to the rice flour of the invention, an additional agent such as a preservative or a desiccant, which can be used to inhibit and/or reduce microbe populations in the rice flour composition, thereby protecting the rice flour composition or products made therefrom from spoilage and allowing for long-term storage of the rice flour composition or products made therefrom.

On the other hand, the rice flour composition may contain additional agents that might be used during the method of preparation of the rice flour, such as water or other manufacturing aids.

In another particular embodiment, the additional agent is a different whitening additive such as titanium dioxide.

In a particular embodiment, the rice flour composition of the invention does not comprise other whitening additives such as titanium dioxide.

In an embodiment, the flours of the invention are comprised in amounts of at least 50%, at least 75, or at least 90% or at least 99.5% weight with respect to the total weight of the rice flour composition. The remaining percentage (making up 100%) can be any of the above mentioned additional agents.

Preferably, the water content in the rice flour composition is 14% or lower, preferably between 14% and 2%, more preferably between 10% and 3%, with respect to the total weight of the rice flour composition. This prevents or lowers microbial contamination of the rice flour upon storage.

Another aspect of the invention refers to the use as a whitening additive of a rice flour according to the present invention as described in any of the above mentioned embodiments, or of a rice flour composition according to the present invention as described in any of the above mentioned embodiments.

In the context of the present invention, a whitening additive refers to a substance which can be incorporated in a product to increase the whiteness of said product. As used herein, the whiteness of a product refers to the extent to which the product diffusely reflects light of all wavelengths throughout the visible spectrum, i.e., the magnitude and uniformity of spectral reflectance. Whiteness may be calculated by a variety of techniques, for instance those determining CIELAB color space, e.g. ISO 11664-4:2008(en).

In an embodiment, the use as a whitening additive of the rice flours or rice flour compositions herein disclosed is in a paper, textile, plastic, paint, sealant, adhesive, concrete, food, fodder, beverage, cosmetic or pharmaceutical product. The use of whitening additives, also referred to as whiteners, whitening agents, or whitening pigments, in these industries is well known in the art.

In a preferred embodiment, the use as a whitening additive is in a food, cosmetic or pharmaceutical product. More preferably, the use as a whitening additive is in a food product, and more particularly in a confectionery product. The use in a confectionery product can be in a candy brittle, a caramel, a chewy candy, a chocolate, carob, a coating, a cotton candy, a fondant, a fudge, a glaze, a gum drop candy, a gummi, a hard boiled candy, a jelly (agar, carrageenan, gelatin, konjac, pectin, or starch based), a licorice, marshmallow, a marzipan, a nougat, a praline, a taffy, a toffee, a sprinkle, or a combination thereof

Similarly, the present invention also provides a paper, textile, plastic, paint, sealant, adhesive, concrete, food, fodder, beverage, cosmetic or pharmaceutical product comprising a rice flour according to the present invention as described in any of the above mentioned embodiments, or a rice flour composition according to the present invention as described in any of the above mentioned embodiments. Preferably, the product is a food, cosmetic or pharmaceutical product. More preferably, the product is a food product, and more particularly it is a confectionery product. The confectionery product can be a candy brittle, a caramel, a chewy candy, a chocolate, carob, a coating, a cotton candy, a fondant, a fudge, a glaze, a gum drop candy, a gummi, a hard boiled candy, a jelly (agar, carrageenan, gelatin, konjac, pectin, or starch based), a licorice, marshmallow, a marzipan, a nougat, a praline, a taffy, a toffee, a sprinkle, or a combination thereof

A food product comprises at least one nutrient. In the context of the present invention, a nutrient is a substance that provides nourishment for the maintenance of life or for growth.

A cosmetic product comprises at least one active cosmetical ingredient. An active cosmetical ingredient is a substance which promotes a desired cosmetic effect. The cosmetic product is preferably a cosmetic composition. The cosmetic product is preferably a facial cream.

A pharmaceutical product comprises at least one active pharmaceutical ingredient (API). An API is a substance which promotes a desired pharmaceutical (i.e. therapeutical) effect. The pharmaceutical product is preferably a pharmaceutical composition. The pharmaceutical product is preferably a dentifrice.

In an embodiment, the flours of the invention are used or comprised in amounts of at least 0.5%, at least 1% or at least 5% weight with respect to the total weight of the product of the invention. In an embodiment, the flours of the invention are used or comprised in amounts of at most 70%, at most 50%, at most 25%, or at most 20% weight with respect to the total weight of the product of the invention. These minimum or maximum values can be combined to generate weight ranges. In preferred embodiments, the flours of the invention are used or comprised in an amount of from 1% to 30%, preferably 1 to 20%, more preferably 5 to 15%, and particularly 10%, by weight with respect to the total weight of the product of the invention.

The products of the invention may comprise other components in addition to the rice flour of the invention. These additional components will vary depending on the type of product. For instance, where the product is a food product, such as a confectionery product, the additional components can be sugars, such as sucrose, xylitol, mannitol, maltitol or sorbitol, preferably sucrose. Other components such as anti-adherent compounds, dispersing agents, film forming agents or binders such as gum arabic; or water, preferably distilled water can also be employed.

In order to afford a better understanding of the invention, the following Examples are presented, it being understood that such Examples are not intended to limit the scope of the invention.

### Examples

### Example 1: Preparation of a rice flour according to the invention

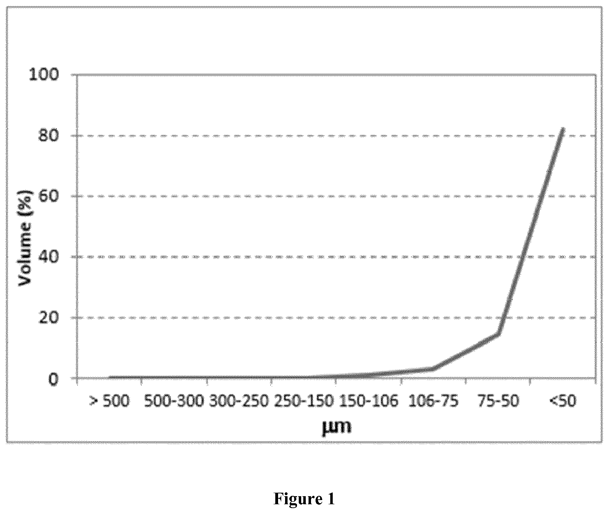

1000 kilograms of Indica rice grains were milled in an industrial air classifier mill (Mikro ACM Air Classifying Mill from Hosokawa). The incoming material selected was dehulled and debranned (non-cooked) rice grains of the Puntal variety. This variety is characterized by large shaped grains having a high amylose content (around 30%). Particles of 50 µmor lower were chosen as the target particle with the highest proportion in the produced flour. After 2 hours of milling, around 945 kilogram of rice flour was obtained. The particle size distribution of this flour was determined by laser diffraction analysis. The results are shown in Table 1 and graphically depicted in Figure 1.

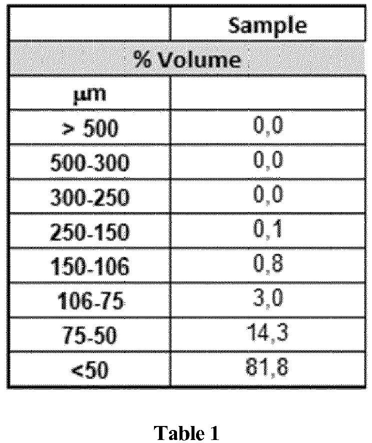

### Example 2: Determination of whiteness in a rice flour product of the invention

A confectionery product comprising the flour of the invention obtained in Example 1 was prepared and compared to corresponding confectionery products comprising either titanium dioxide or purified rice starch.

The composition of the products was as follows (values are weight percentages of each component with respect to the total weight of the food product):

| | **Titanium dioxide** | **Rice starch** | **Micronized rice flour** |
|---|---|---|---|
| Sugar | 60 | 60 | 60 |
| Titanium dioxide | 1 | | |
| Purified rice starch | | 10 | |
| Micronized rice flour* | | | 10 |
| Distilled water | 39 | 30 | 30 |

These mixtures were heated for 5 minutes at 60°C, stirred, and then poured into a petri dish. The content of the petri dishes were air dried until a solid slurry was obtained.

The color was measured by the determination of the CIELab parameters with a Minolta CR-210 colorimeter.

The following results were observed:

| | **L** | **a** | **b** |
|---|---|---|---|
| Titanium dioxide | 90.6 | -0.4 | -0.3 |
| Purified rice starch | 84.5 | 0.1 | -0.9 |
| Micronized rice flour | 85.3 | 0.1 | 0.5 |

The use of a flour according to the present invention gave nice and white glazes. As can be seen from the above table, the rice flour of the invention performed as a whitener with very similar results to rice starch or titanium dioxide.

## Claims

1. A rice flour formed by rice flour particles, wherein rice flour particles having a size of between 10 and 100 µm represent at least 50% volume of the rice flour particles, and wherein the weight of amylose in the rice flour is at least 20% by weight with respect to the total weight of the rice flour.

2. Rice flour according to claim 1, wherein rice flour particles having a size of between 20 and 75 µm represent at least 90% volume of the rice flour particles.

3. Rice flour according to any one of claims 1 to 2, wherein the weight of amylose in the rice flour is between 20% and 35% by weight with respect to the total weight of the rice flour.

4. Rice flour according to any one of claims 1 to 3, wherein at least 80% of the starch in the rice flour is undamaged as determined by AACCI method 76-30.02.

5. A method for preparing a rice flour as defined in any one of claims 1 to 4, comprising the steps of:
a) selecting a starting rice flour wherein the weight of amylose in the rice flour is at least 20% by weight with respect to the total weight of the rice flour;
b) reducing the particle size of the starting rice flour of step (a) until a rice flour formed by rice flour particles is obtained wherein rice flour particles having a size of between 10 and 100 µm represent at least 50% volume of the rice flour particles, and wherein the weight of amylose in the rice flour is at least 20% by weight with respect to the total weight of the rice flour.

6. A method for preparing a rice flour as defined in any one of claims 1 to 4, comprising the steps of:
a) selecting rice grains wherein the weight of amylose in the rice grains is at least 20% by weight with respect to the total weight of the rice grains;
b) reducing the particle size of the rice grains of step (a) until a rice flour formed by rice flour particles is obtained wherein rice flour particles having a size of between 10 and 100 µm represent at least 50% volume of the rice flour particles, and wherein the weight of amylose in the rice flour is at least 20% by weight with respect to the total weight of the rice flour.

7. Method according to any one of claims 5 or 6, further comprising a step c) wherein the flour obtained in step b) is subjected to heating.

8. Method according to any one of claims 5 to 7, wherein step b) comprises grinding the rice grains or starting rice flour of step a) to produce ground rice grains or ground starting rice flour particles; followed by air classification of the ground rice grains or ground starting rice flour particles.

9. Method according to claim 8, wherein the grinding and the air classification are carried out in an air classifier.

10. A rice flour composition comprising a rice flour as defined in any one of claims 1 to 4.

11. A paper, textile, plastic, paint, sealant, adhesive, concrete, food, fodder, beverage, cosmetic or pharmaceutical product comprising a rice flour as defined in any one of claims 1 to 4, or comprising a rice flour composition as defined in claim 10.

12. A food product, preferably a confectionery product, comprising a rice flour as defined in any one of claims 1 to 4, or comprising a rice flour composition as defined in claim 10.

13. A candy brittle, a caramel, a chewy candy, a chocolate, carob, a coating, a cotton candy, a fondant, a fudge, a glaze, a gum drop candy, a gummi, a hard boiled candy, a jelly, a licorice, a marshmallow, a marzipan, a nougat, a praline, a taffy, a toffee, a sprinkle, comprising a rice flour as defined in any one of claims 1 to 4, or comprising a rice flour composition as defined in claim 10.

14. Product according to any one of claims 11 to 13, wherein the rice flour is present in an amount of from 0.5 to 70% weight with respect to the total weight of the product.

15. Use of a rice flour as defined in any one of claims 1 to 4, or of a rice flour composition as defined in claim 10, as a whitening additive.
